# EUROPEAN PATENT SPECIFICATION

(11) **EP 1 933 746 B1**
(45) Date of publication and mention of the grant of the patent: **17.03.2010**
(21) Application number: 06765091.1
(22) Date of filing: 24.07.2006
(51) Int. Cl.: A61B 18/14

(54) **ELECTRICAL CONNECTOR FOR ELECTRO-SURGICAL INSTRUMENT**
ELEKTRISCHES VERBINDUNGSGLIED FÜR EIN ELEKTROCHIRURGISCHES INSTRUMENT
CONNECTEUR ÉLECTRIQUE POUR INSTRUMENT ÉLECTRO-CHIRURGICAL

(30) Priority: 22.07.2005 GB 0515050
(43) Date of publication of application: 25.06.2008
(73) Proprietor: Brooke, Gerard, Stroud, Gloucestershire GL5 3TJ (GB)
(72) Inventor: Brooke, Gerard, Stroud, Gloucestershire GL5 3TJ (GB)
(74) Representative: Newell, William Joseph
(86) International application number: PCT/GB2006/002765
(87) International publication number: WO 2007/010292

(56) References cited:
- DE-A1- 19 513 588
- US-A- 5 780 993
- US-A- 6 105 581
- US-B1- 6 475 040

## Description

This invention relates to an electrical connector for connection to electro-surgical instruments and in particular, but not exclusively, to bipolar forceps.

In a bipolar electro-surgical instrument the current source is connected to the instrument by a two core cable and the instrument has two electrically conducting portions (forcep tips) across which a circuit is made when the tips contact tissue. The cable is conventionally connected to the instrument by means of a push fit female connector which connects with male terminals on the instrument. There are two different types of male connector on the market, one type prevailing in UK/Europe and the other prevailing in USA, but both are in common usage. In UK/Europe, the common form of male terminal comprises two spaced parallel spades and the UK/European connector comprises a flat or round plug which pushes on to make the electrical connection. In the USA however, the common plug comprises two spaced pins and so the connector comprises two spaced bores which push fit on to the pins.

Depending on the particular surgeon's preferences, hospital may have instruments of both types in use and so at present it is necessary to stock two different types of cables with connectors. This is problematic both in terms of stock levels and also the risk of getting the wrong connector for a particular instrument. Document US-A-6 105 581 is regarded as being the most relevant prior art. A connector for an electro-surgical instrument is shown. The connector comprises a plurality of parallel elongated conducting elements having parallel bores.

I have therefore developed an electrical connector capable of connecting to either type of plug by a simple push fit. The invention is defined in claim 1.

Preferably, the exposed generally parallel surface regions face each other and are located one to either side of a recess formed in said body portion.

Preferably, each electrically conducting element is capable of resilient movement within said body. The resilient movement serves both to ensure good electrical connection with the terminals of the first type of plug but also slight adjustments in spacing of the electrically conducting elements according to the particular connection.

Preferably, the electrically conducting elements are secured to the limbs of a U- or Y-shaped holder. The holder may also be connected to the cable in use. Preferably the holder is formed of a plastics material and conveniently may be over-moulded onto the electrically conducting elements and to the cable.

Preferably said body portion comprises an outer sleeve element generally surrounding the holder. Preferably the outer sleeve material is formed of an elastomeric material. Preferably the outer sleeve has portions of reduced wall thickness adapted to assist resilient movement of the elongate electrically conducting elements within said body.

The invention may be performed in various ways, and an embodiment thereof will now be described by way of example only, reference being made to the accompanying drawings in which:
Figure 1 is a general perspective view of a dual purpose bipolar connector in accordance with this invention;
Figure 2 is a perspective view of a sub-assembly of the connector of figure 1;
Figure 3 is a longitudinal sectional view through the sub-assembly Figure . 2;
Figure 4 is a longitudinal view similar to Figure 3 but with the sleeve over-moulded to make up the connector;
Figure 5 is an end view on the connector of Figure 1, and
Figures 6 (a) and (b) are respective end views on a UK/European style plug and a US style plug respectively.

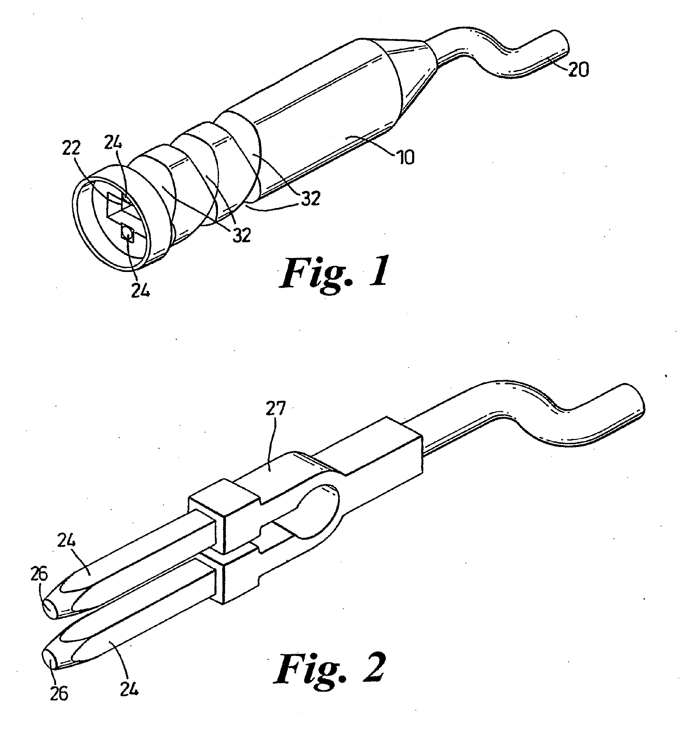

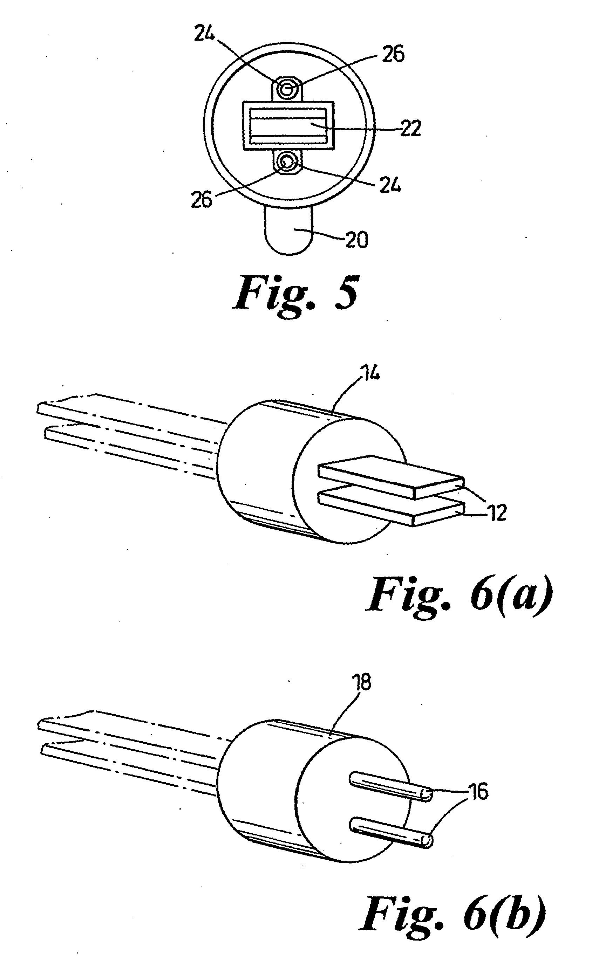

Referring initially to Figure 1, the connector 10 illustrated therein is intended to be connectable to either a UK/European style plug or a US style plug shown in Figures 6 (a) and (b). The UK/European plug comprises two parallel space spade terminals 12 projecting from a connector block 14. By contrast the US style plug comprises two pins 16 projecting from a connector block 18.

The connector 10 is connected at one end to a two core cable 20 and has a connecting region at its other end. The connecting region comprises a recess 22 to either side of which are located brass elongate contacts 24 having bores 26. The recess 22 is designed so that the spades 12 of the UK/European style plug can be slid therein with the spades making electrical contact with the contacts 24. Furthermore, the spacing of the contacts 24 is such that their bores are in a position to receive the pins 16 of a US style plug.

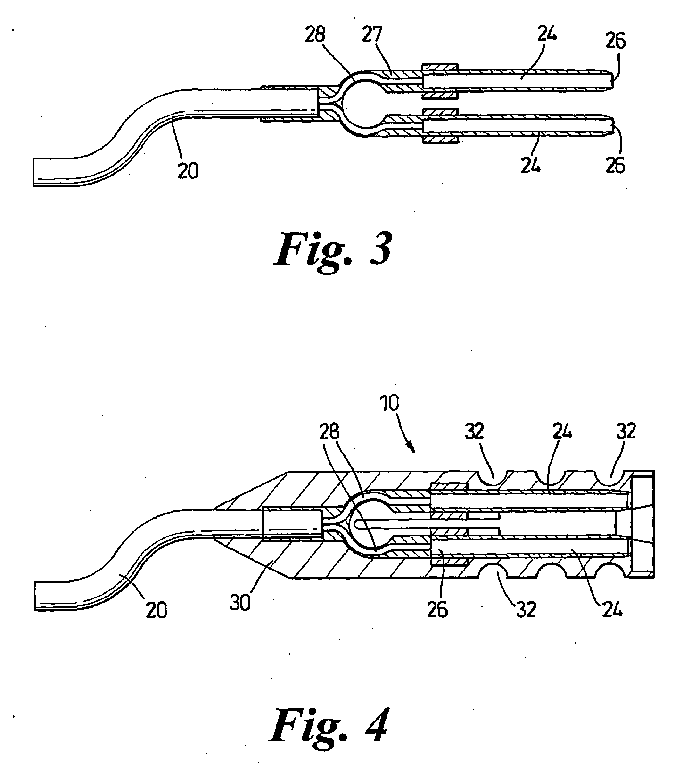

Referring now to Figures 2 and 3, the connector is made up of an assembly comprising the contacts 22 and an over-moulded bifurcated holder 27 which is over-moulded onto the proximal ends of the contacts 24 and to the sheath of the cable 20, with the twin cores 28 embedded in the moulding. The contact holder is preferably moulded of a material such as polypropylene and its material qualities and the shape of the support are such to allow resilient flexing movement of the contacts relative to each other.

Referring now to Figure 4, an elastomeric outer sleeve 30 is over-moulded onto the assembly of Figure 2 to provide an outer cover whilst leaving the recess 22 and the bores in the contacts 24 exposed. As seen in Figures 1 and 4, the sleeve 30 has regions 32 of reduced wall thickness to allow outward flexing of the electrical contacts.

## Claims

1. An electrical connector (10) for connection to at least two different types of electro-surgical instrument, said electrical connector comprising a body portion, and two elongate electrically conducting elements (24) mounted in said body in generally parallel spaced relationship leaving spaced generally paralle external surface regions of said elongate electrically conducting elements exposed, each said elongate element having an internal bore (26), the size and location of said bores and the spacing and disposition of said exposed regions being such that the electrical connector may in use be push fitted to a first type of plug comprising two spaced spade terminals to make respective electrical connections between said spaced terminals and said exposed regions, or to a second type of plug comprising two spaced parallel pins which enter respective bores in said elongate electrically conducting elements to make respective electrical connections between said pins and said elongate electrically conducting elements.

2. An electrical connector according to claim 1 wherein said exposed generally parallel surface regions face each other and are located one to either side of a recess formed in said body portion.

3. An electrical connector according to claim 1 or claim 2 wherein at least one of said electrically conducting elements is capable of resilient movement within said body.

4. An electrical connector according to any of the preceding claims, wherein the electrically conducting elements are secured to the limbs of a U or Y shaped holder.

5. An electrical connector according to claim 4 wherein the holder is formed of a plastics material.

6. An electrical connector according to claim 4 or claim 5 wherein said holder is made by over moulding onto the electrically conducting elements and to a connection cable.

7. An electrical connector according to any of claims 4 to 6 wherein said body portion comprises an outer sleeve element generally surrounding the holder.

8. An electrical connector according to claim 7 wherein said outer sleeve is formed of an elastomeric material.

9. An electrical connector according to claim 7 or claim 8 wherein said outer sleeve has portions of reduced wall thickness adapted to assist resilient movement of the elongate electrically conducting elements within said body.

## Patentansprüche

1. Elektrisches Verbindungsglied (10) zur Verbindung von wenigstens zwei unterschiedlichen Arten eines elektro-chirurgischen Instruments, wobei das elektrische Verbindungsglied folgende Elemente umfaßt: einen Körperteil und zwei längliche, elektrisch leitende Elemente (24), die in dem Körper im allgemeinen parallel mit Abstand angeordnet sind, so daß beabstandete, im allgemeinen parallele externe Oberflächenbereiche der länglichen, elektrisch leitenden Elemente freiliegen, wobei jedes längliche Element eine innere Bohrung (26) aufweist, Größe und Ort dieser Bohrungen und der Abstand sowie die Anordnung der freiliegenden Bereiche derart sind, daß das elektrische Verbindungsglied bei Benutzung in eine erste Art Stecker gedrückt werden kann, umfassend zwei mit Abstand getrennte Kabelschuhe zur Bildung entsprechender elektrischer Anschlüsse zwischen den mit Abstand getrennten Kabelschuhen und den genannten freiliegenden Bereichen, oder eine zweite Art Stecker, umfassend zwei mit Abstand getrennte parallele Stifte, die in entsprechende Bohrungen in den länglichen, elektrisch leitenden Elementen eintreten, um zwischen den Stiften und den länglichen, elektrisch leitenden Elementen entsprechende elektrische Verbindungen herzustellen.

2. Elektrisches Verbindungsglied nach Anspruch 1, **dadurch gekennzeichnet, daß** die freiliegenden, im allgemeinen parallelen Oberflächenbereiche einander zugewendet sind und auf jeder Seite einer Vertiefung liegen, die in dem genannten Körperteil ausgebildet ist.

3. Elektrisches Verbindungsglied nach Anspruch 1 oder 2, **dadurch gekennzeichnet, daß** wenigstens eines der elektrisch leitenden Elemente in der Lage ist, sich in dem Körper federnd zu bewegen.

4. Elektrisches Verbindungsglied nach einem der vorhergehenden Ansprüche, **dadurch gekennzeichnet, daß** die elektrisch leitenden Elemente an den Schenkeln eines U- oder Y-förmigen Halters befestigt sind.

5. Elektrisches Verbindungsglied nach Anspruch 4, **dadurch gekennzeichnet, daß** der Halter aus einem Kunststoffmaterial besteht.

6. Elektrisches Verbindungsglied nach Anspruch 4 oder 5, **dadurch gekennzeichnet, daß** der Halter durch Spritzgießen auf die elektrisch leitenden Elemente und ein Anschlußkabel hergestellt ist.

7. Elektrisches Verbindungsglied nach einem der Ansprüche 4 bis 6, **dadurch gekennzeichnet, daß** der Körperteil ein äußeres Hülsenelement aufweist, das den Halter ganz allgemein umgibt.

8. Elektrisches Verbindungsglied nach Anspruch 7, **dadurch gekennzeichnet, daß** die äußere Hülse aus einem Elastomermaterial geformt ist.

9. Elektrisches Verbindungsglied nach Anspruch 7 oder 8, **dadurch gekennzeichnet, daß** die äußere Hülse Abschnitte verringerter BWanddicke aufweist, die in der Lage sind, die federnde Bewegung der länglichen, elektrisch leitenden Elemente in dem Körper zu unterstützen.

## Revendications

1. Connecteur électrique (10) pour le raccordement à au moins deux types différents d'instrument électro-chirurgical, ledit connecteur électrique comprenant une partie de corps, et deux éléments électriquement conducteurs allongés (24) montés dans ledit corps en relation espacée généralement parallèle, laissant exposées des régions de surface externe généralement parallèles espacées desdits éléments électriquement conducteurs allongés, chaque élément allongé précité ayant un alésage interne (26), la dimension et la position desdits alésages et l'espacement et la disposition desdites régions exposées étant tels que le connecteur électrique peut, lors de l'utilisation, être ajusté gras dans un premier type de fiche comprenant deux bornes embrochables espacées pour établir des contacts électriques respectifs entre lesdites bornes espacées et lesdites régions exposées, ou dans un second type de fiche comprenant deux broches parallèles espacées qui entrent dans des alésages respectifs dans lesdits éléments électriquement conducteurs allongés pour établir des contacts électriques respectifs entre lesdites broches et lesdits éléments électriquement conducteurs allongés.

2. Connecteur électrique selon la revendication 1, dans lequel lesdites régions de surface généralement parallèles exposées sont tournées l'une vers l'autre et sont situées chacune sur un côté d'une cavité formée dans ladite partie de corps.

3. Connecteur électrique selon la revendication 1 ou la revendication 2, dans lequel au moins l'un desdits éléments électriquement conducteurs est capable d'un mouvement élastique à l'intérieur dudit corps.

4. Connecteur électrique selon l'une quelconque des revendications précédentes, dans lequel les éléments électriquement conducteurs sont fixés aux branches d'un support en forme de U ou de Y.

5. Connecteur électrique selon la revendication 4, dans lequel le support est formé d'une matière plastique.

6. Connecteur électrique selon la revendication 4 ou la revendication 5, dans lequel ledit support est réalisé par surmoulage sur les éléments électriquement conducteurs et sur un câble de connexion.

7. Connecteur électrique selon l'une quelconque des revendications 4 à 6, dans lequel ladite partie de corps comprend un élément manchon externe entourant généralement le support.

8. Connecteur électrique selon la revendication 7, dans lequel ledit manchon externe est formé d'un matériau élastomère.

9. Connecteur électrique selon la revendication 7 ou la revendication 8, dans lequel ledit manchon externe possède des parties d'épaisseur de paroi réduite aptes à aider le mouvement élastique des éléments électriquement conducteurs allongés à l'intérieur dudit corps.
